Europäisches Patentamt

European Patent Office

Office européen des brevets

(11) Numéro de publication: **0 363 239**
**A2**

## DEMANDE DE BREVET EUROPEEN

(21) Numéro de dépôt: 89402529.5

(51) Int. Cl.5: **A61B 17/22**

(22) Date de dépôt: **15.09.89**

(30) Priorité: **06.10.88 FR 8813094**

(43) Date de publication de la demande:
**11.04.90 Bulletin 90/15**

(84) Etats contractants désignés:
**AT BE CH DE ES FR GB GR IT LI NL SE**

(71) Demandeur: **EDAP INTERNATIONAL**
**Z.I. le Parc aux Vignes Rue des Vieilles**
**Vignes**
**F-77200 Croissy Beaubourg(FR)**

(72) Inventeur: **Dory, Jacques**
**91, rue des Molveaux**
**F-77450 Coupvray(FR)**

(74) Mandataire: **Marquer, Francis et al**
**Cabinet Moutard 35, Avenue Victor Hugo**
**F-78960 Voisins le Bretonneux(FR)**

(54) **Appareil de destruction localisée de structures molles au moyen d'ondes élastiques de pression négative.**

(57) L'appareil comporte une source piézoélectrique, répartie sur une surface d'oscillations élastiques brèves comportant des crêtes négatives et des moyens de focaliser les oscillations sur la cible. La fréquence des oscillations est réglée entre 0,3 et 10 MHz et la durée des trains d'ondes, à des valeurs comprises entre 1 μs et 1 ms. La cadence des tirs est comprise entre quelques Hz et 2 ou 3 KHz.

EP 0 363 239 A2

## APPAREIL DE DESTRUCTION LOCALISEE DE STRUCTURES MOLLES AU MOYEN D'ONDES ELASTIQUES DE PRESSION NEGATIVE.

L'invention a pour objet un appareil destiné à la destruction d'une cible constituée par des structures molles et localisées au sein d'un milieu qui empêche d'y accéder directement par des moyens mécaniques.

Parmi les applications d'un tel appareil, on peut citer la destruction de cellules vivantes, par exemple de cellules tumorales, à l'intérieur du corps d'un patient, ou la coloration d'une matière dans sa masse en y faisant exploser des microbilles aptes à libérer des colorants, ou la mise en oeuvre localisée d'une réaction chimique, par libération de matières susceptibles d'entrer en réaction contenues dans des microbilles.

La destruction, par des moyens extracorporels, d'une cible constituée par des structures mécaniquement résistantes, s'effectue actuellement au moyen d'ondes élastiques de pression positive qui soumettent lesdites structures à des efforts de compression.

A titre d'exemple, dans les lithotripteurs connus, des impulsions élastiques d'une durée de l'ordre de la microseconde, focalisées sur un calcul pulvérisent celui-ci.

Plus particulièrement, dans les lithotripteurs à source émettrice répartie sur une surface et du type piézoélectrique, ces ondes élastiques, à moins que des dispositions très spécifiques soient prises, comportent des alternances de pression positive (par rapport à la pression moyenne de l'oscillation) et des alternances négatives. Toutefois, il est connu que ces dernières ne contribuent pas à la destruction des calculs et ont par contre l'effet nuisible de provoquer la formation d'une barrière composée de bulles engendrées par cavitation et qui gêne la propagation du faisceau, pouvant même aller jusqu'à l'interrompre.

L'art antérieur relatif à la destruction de cibles molles est par exemple constitué par l'hyperthermie, c'est-à-dire l'échauffement localisé à des températures bien déterminées. A cet effet, on utilise des trains d'ondes élastiques focalisées sur la cible. Ces trains d'ondes peuvent être engendrés (comme en lithotripsie) par une source émettrice répartie sur une surface et du type piézoélectrique, mais, alors qu'en lithotripsie on utilise en général des impulsions ayant une durée très brève (de l'ordre de la microseconde) et des puissances de crête considérables (de l'ordre de plusieurs dizaines de kilowatts), en hyperthermie, les puissances sont réduites à des valeurs de crête de 10 à 100 Watts par exemple et les trains d'ondes sont émis pendant des durées beaucoup plus longues (pouvant atteindre la seconde par exemple), suffisantes pour provoquer l'échauffement. Dans le cas de l'hyperthermie, les fréquences sont de l'ordre de 0,5 à 5 MHz.

L'invention part de la découverte du fait que les structures molles sont susceptibles d'être détruites efficacement au moyen d'ondes de pression négatives de brève durée, par des effets de traction qui les déchirent.

L'appareil de destruction localisée de structures molles suivant l'invention est principalement caractérisé en ce qu'il comporte une source de trains d'oscillations élastiques brèves répartie sur une surface et du type piézoélectrique, des moyens de focaliser un faisceau de telles oscillations sur une cible et des moyens de régler la fréquence des oscillations à des valeurs comprises entre 0,3 et 10 MHz, des moyens de régler la durée des trains d'ondes à des valeurs prédéterminées, avantageusement comprises entre 1 $\mu$s et 1 ms et des moyens de régler la cadence d'émission des trains (cadence des tirs) prédéterminée avantageusement à des valeurs comprises entre quelques Hz et 2 ou 3 KHz.

Suivant un mode d'exécution préféré, l'appareil est agencé pour que lesdites oscillations comportent des crêtes négatives prépondérantes, c'est-à-dire d'amplitude grande vis-à-vis de celle de leurs crêtes positives.

Suivant une particularité avantageuse, l'appareil comporte des moyens de régler la puissance d'émission préalablement aux tirs en focalisant le faisceau sur la cible et en observant, à l'aide desdits moyens échographiques, l'apparition d'échos par réflexion sur la cible et en augmentant la puissance d'émission jusqu'à un seuil pour lequel on constate la disparition des échos sur la cible et/ou l'apparition d'échos spécifiques signalant que le seuil de cavitation est atteint, la puissance retenue pour le réglage final étant choisie en deçà dudit seuil, de préférence au voisinage de celui-ci.

La disparition des échos correspond en effet à l'apparition du phénomène de cavitation dans la région du foyer, et la puissance ainsi réglée sera donc la plus efficace pour la destruction des cellules tumorales.

D'autres particularités, ainsi que les avantages de l'invention apparaîtront clairement à la lumière de la description ci-après donnée à titre d'exemple.

Exemple

On utilise un lithotripteur du type piézoélectri-

2

que à source d'émission répartie sur une surface, avantageusement du genre décrit dans le brevet FR No 2 556 582 déposé le 14 Décembre 1983.

Pour obtenir une prépondérance des ondes ultrasonores négatives, il suffit par exemple de changer le sens des enroulements du transformateur d'excitation du transducteur céramique de puissance, de façon à l'exciter avec des trains d'impulsions où les alternances négatives sont prépondérantes.

On notera qu'un lithotripteur du type électrohydraulique, c'est-à-dire à trajet d'étincelles dans un liquide de couplage, n'est pas susceptible d'engendrer des impulsions négatives et ne serait donc pas utilisable.

La forme d'onde, telle qu'elle est définie par celle du signal électrique d'excitation du transducteur, consiste en oscillations de fréquence avantageusement comprise entre 300 KHz et 1 MHz pour la desctruction de tissus profonds, et entre 5 et 10 MHz pour la destruction de tissus superficiels. Les trains d'ondes ou "tirs" ont par exemple des durées comprises entre 1 μs et 1 ms et la cadence des tirs est par exemple, comprise entre quelques Hz et 2 ou 3 KHz, ces valeurs nétant pas limitatives. Les cadences les plus faibles sont celles qui donnent la meilleure image échographique de la cible. Les durées de trains d'ondes sont avantageusement déterminées pour que la destruction des cellules s'effectue principalement par contrainte mécanique : des durées plus grandes entraîneraient l'apparition d'un phénomène d'hyperthermie.

Le procédé comporte une phase préalable de réglage de la puissance d'émission du transducteur principal, au cours de laquelle la puissance de crête des oscillations est d'abord réglée à une faible valeur (quelques watts par exemple), tandis que la durée des trains d'ondes est elle-même réglée à sa valeur minimale et leur cadence à sa valeur maximum. Les échos provenant de la réflexion sur la cible des impulsions négatives émises par le transducteur de puissance sont captés par le transducteur auxiliaire d'échographie du lithotripteur, permettent la visualisation de la distribution d'énergie dans la tache focale, selon le procédé décrit dans le brevet susvisé. On augmente ensuite progressivement la puissance d'émission, jusqu'à la disparition des échos sur la cible : il apparaît alors en outre généralement, sur l'écran de visualisation, un petit nuage brillant qui indique l'apparition du phénomène de cavitation au foyer.

Si l'on augmentait encore la puissance, la cavitation se développerait entre la surface d'émission et le foyer, qui ne serait plus irradié.

On règle la puissance finale un peu en deçà du seuil de disparition des échos situés dans la zone focale et on procède ensuite au réglage approprié de la durée et de la cadence des trains d'ondes.

A titre d'exemple, la puissance de crête retenue sera de l'ordre de quelques kilowatts, correspondant à des pressions de crête de l'ordre de 50 à 60 bars.

Les oscillations négatives utilisées agissent principalement par contrainte mécanique, en exerçant des effets de traction qui déchirent les cellules (alors que les structures molles résistent beaucoup mieux aux effets de compression engendrés par des oscillations positives).

L'exécution pratique des réglages de puissance, de durée et de cadences de trains d'ondes, ainsi que l'obtention de la polarité appropriée des oscillations d'excitation du transducteur sont à la portée de l'homme du métier et pourront se faire de différentes manières sans s'écarter de l'esprit de l'invention.

A titre d'exemple, l'apparition de la cavitation dans la région focale pourrait être décelée en utilisant une échographie du type A et en observant la déformation des échos en fonction de la puissance d'émission.

## Revendications

1. Appareil de destruction localisée de structures molles, caractérisé en ce qu'il comporte une source d'oscillations élastiques brèves comportant des composantes négatives, répartie sur une surface et du type piézoélectrique, des moyens de focaliser un faisceau de telles oscillations sur une cible et des moyens de régler la fréquence des oscillations à des valeurs comprises entre 0,3 et 10 MHz, des moyens de régler la durée des trains d'ondes à des valeurs prédéterminées, avantageusement comprises entre 1 μs et 1 ms et des moyens de régler la cadence d'émission des trains (cadence des tirs) à des valeurs prédéterminées avantageusement comprises entre quelques Hz et 2 ou 3 KHz.

2. Appareil selon la revendication 1, caractérisé en ce qu'il est agencé pour que lesdites oscillations comportent des crêtes négatives prépondérantes, c'est-à-dire d'amplitude grande vis-à-vis de celle de leurs crêtes positives.

3. Appareil selon la revendication 1 ou 2, caractérisé en ce qu'il comporte des moyens de régler la puissance d'émission préalablement aux tirs en focalisant le faisceau sur la cible et en observant, à l'aide desdits moyens échographiques, l'apparition d'échos par réflexion sur la cible et en augmentant la puissance d'émission jusqu'à un seuil pour lequel l'on constate la disparition des échos sur la cible et/ou l'apparition d'échos spécifiques signalant que le seuil de cavitation est atteint, la puissance retenue pour le réglage final étant choisie en deçà dudit seuil, de préférence au voisinage de celui-ci.

4. Appareil selon l'une des revendications 1 à 3, caractérisé en ce que la puissance d'émission est réglée à une valeur de crête de l'ordre de quelques kilowatts.